# EUROPEAN PATENT SPECIFICATION

(11) **EP 1 338 303 B1**
(45) Date of publication and mention of the grant of the patent: **05.10.2005**
(21) Application number: 02425093.8
(22) Date of filing: 20.02.2002
(51) Int. Cl.: A61P 43/00, A61K 35/78

(54) **Detoxifying phytotherapeutic composition from two plant extracts and process for preparing the same**
Entgiftende phytotherapeutische Zusammensetzung aus zwei Pflanzenextrakten und Verfahren zu deren Herstellung
Composition phytothérapeutique détoxifiante comprenant deux extraits de plantes, et procédé pour sa préparation

(43) Date of publication of application: 27.08.2003
(73) Proprietor: Kilb, Katrin, 21028 Sesto Calende VA (IT); Mariani, Franca, 20064 Gorgonzola MI (IT)
(72) Inventor: Kilb, Katrin, 21028 Sesto Calende VA (IT); Mariani, Franca, 20064 Gorgonzola MI (IT)
(74) Representative: Pizzoli, Antonio

(56) References cited:
- WO-A-98/40086
- US-A- 5 595 743
- DATABASE WPI Week 200164 Derwent Publications Ltd., London, GB; AN 2001-569529 XP002212350 & KR 2001 028 473 A (SHIN J C), 4 June 2001 (2001-06-04)
- DATABASE WPI Week 198935 Derwent Publications Ltd., London, GB; AN 1989-251055 XP002212351 & HU 48 819 A (SEMMELWEIS ORVOSTUD), 28 July 1989 (1989-07-28)
- DATABASE EPODOC [Online] EUROPEAN PATENT OFFICE, THE HAGUE, NL; XP002212349 & KR 9 007 544 B (KIM MYUNG-HO) 15 October 1990 (1990-10-15)

## Description

The present invention relates to a detoxifying phytotherapic composition and to a process for preparing the same.

It is known that the human organism may contract intoxications due either to toxic substances ingested or anyway absorbed from the outside, or to an accumulation of substances produced by the organism itself, especially following to infectious diseases, and not eliminated in time.

Intoxications, which constitute real pathological conditions, may derive for example from a pharmacological therapy protracted in time, such as a therapy with antibiotics, chemotherapeutics, sulfonamides, or an antiviral or antiretroviral therapy.

As a matter of fact, it is known that metabolization of drugs by the organism brings about the formation of scoriae or toxins which have to be eliminated. However, in order to be efficacious, drugs must often be assumed in very high doses and consequently the toxins produced are not completely eliminated by the organism, thus being accumulated inside the organism itself. Said accumulation may cause in the long term serious damage to the organs, particularly to the liver, and may additionally damage the cell, particularly at mitochondrial level.

Other serious intoxications may be caused for instance by ingestion of poisons of various nature or by drug abuse. Another possible cause of intoxications and cellular disorders are ionizing radiations.

Efficacious remedies for purifying the organism by freeing it from an accumulation of toxins and for favoring a situation of equilibrium also at the cellular level are not presently known.

KR2001028473 discloses a peach wine composition obtained by fermentation in a closed container of peaches (by weight, 50-60%), sugar (30%), mulberry leaves (5%) and wormwood (5%). The composition is described to have beneficial effects in improving geriatric diseases such as diabetes and gastrointestinal ailments.

HU48819 discloses a therapeutic composition useful for the treatment of genetic mucoviscidosis comprising rape honey as the main ingredient, and minor quantities of elder, lime blossoms, acacia and powdered blood-wort, cowslip-root wormwood, mulberry leaves and cinnamon, and pounded garlic.

However, the described beneficial effects of these compositions are due to the combination of many active ingredients and do not include the detoxification of the organism.

Object of the present invention is therefore to provide a detoxifying composition and a process for preparing it. Said object is achieved with a phytotherapic detoxifying composition whose main features are specified in claim 1 and a process for the preparation thereof whose main features are specified in claim 7. Other features of the phytotherapic composition according to the present invention and of the process for the preparation thereof are specified in the remaining claims.

A first advantage of the phytotherapic composition according to the present invention consists in the efficacy thereof in the detoxification of the human or animal organisms from any kind of toxins. Said composition may therefore be administered to patients who have been subjected to pharmacological therapies of various type, for example with antibiotics, chemotherapeutics or sulfonamides, or to antiviral or antiretroviral therapy, and in case of presence of HIV serum antibodies. In addition, the phytotherapic composition according to the present invention may help those who used drugs or those who are being subjected to ionizing radiations or have been subjected thereto.

Further advantages and features of the phytotherapic composition according to the present invention and of the process for the production thereof will appear to those which are skilled in the art from the following detailed description of an embodiment thereof.

The phytotherapic composition according to the present invention comprises the extracts of two medicinal plants, and in particular the extracts of Artemisia Absinthium L. and Morus Nigra L.

The first plant, better known under the vulgar name of absinthium, is a herbaceous plant from which absinthin is derived, which is a bitter substance having an intense smell and tonic, stimulating properties. Its vermifugal, antipyretic, anti-depressant and anti-anorexic properties are also known.

The second plant, known as mulberry, is known for its hypoglycemic property.

It has been now surprisingly found that the extracts of these two plants develop a synergic activity with detoxifying effect if they are administered simultaneously to an individual. Said effect was completely unpredictable on the base of the above mentioned properties of the single plants.

The extracts to be used in the phytotherapic composition according to the present invention can be obtained starting from leaves of Morus Nigra L. and from blooming tops with leaves of Artemisia Absinthium L., which have been suitably dried and processed in tisane-cut or in powder, by using an extraction process known for the preparation of extracts of other medicinal plants.

Said extracts may be obtained separately and then mixed in the desired proportions, but preferably they are prepared by a single extraction process starting from a mixture comprising Morus Nigra L. and Artemisia Absinthium L.

Preferably, the weight percentage of Morus Nigra L. in said mixture is within about 52% and 63% and that of Artemisia Absinthium L. is within about 37% and 48% by weight.

It has been experimentally found that the beneficial effect of the phytotherapic composition according to the present invention are maximum when the extracts of the two medicinal plants are obtained starting from a mixture wherein the weight percentage of Moms Nigra L. is about 57% and the weight percentage of Artemisia Absinthium L. is about 43%.

Further to the extracts of the two plants, the phytotherapic composition according to the present invention advantageously comprises a pharmaceutically acceptable excipient. Preferably, said extract is dissolved in a hydroalcoholic solution, for example of concentration of about 60%.

The composition according to the present invention may be administered orally and is efficacious in very small quantities, for example it can be advised to swallow one drop thereof diluted in some water. Anyway, the modalities of administration depend from the health conditions of the patients and on other factors such as age, sex and body weight.

The composition according to the present invention can be prepared by first providing a mixture comprising leaves of Morus Nigra L. and blooming tops with leaves of Artemisia Absinthium L., which have been suitably dried and processed in tisane-cut or in powder, in the above indicated preferred proportions.

The so prepared mixture is then allowed to macerate in a hydroalcoholic solution at room temperature in a container of suitable material, for example stainless steel. Preferably, the weight ratio between said mixture and the hydroalcoholic solution is about 1 : 10.

The mixture is then allowed to macerate for at least 2 weeks, and finally it is filtered. After filtration, the phytotherapic composition according to the present invention may be packaged and administered to patients in the above indicated modalities.

## Claims

1. A phytotherapic composition **characterized in that** it comprises, as active ingredients, extracts of Morus Nigra L. and Artemisia Absinthium L. only.

2. A phytotherapic composition according to claim 1, **characterized in that** said extracts are obtainable starting from a single mixture comprising Morus Nigra L. and Artemisia Absinthium L.

3. A phytotherapic composition according to claim 2, **characterized in that** said mixture comprises Moms Nigra L. in percentage within 52% and 63% by weight and Artemisia Absinthium L. in percentage within 37% and 48% by weight.

4. A composition according to claim 3, **characterized in that** said mixture comprises Morus Nigra L. in weight percentage of 57% and Artemisia Absinthium in weight percentage of 43%.

5. A phytotherapic composition according to one or more of the preceding claims, **characterized in that** said extracts are dissolved in a hydroalcoholic solution.

6. A phytotherapic composition according to claim 5, **characterized in that** said hydroalcoholic composition has a concentration of 60%.

7. A process for the production of a phytotherapic composition according to one or more of the preceding claims, comprising the steps of:
- preparing a mixture comprising Morus Nigra L. and Artemisia Absinthium L.;
- macerating said mixture in a hydroalcoholic solution;
- filtrating the mixture.

8. A process according to claim 7, **characterized in that** in the maceration step the weight ratio between said mixture and said hydroalcoholic solution is 1:10.

9. A process according to claims 7 or 8, **characterized in that** said maceration step is carried out at room temperature.

10. A process according to claim 9, **characterized in that** the duration of said maceration step is at least 2 weeks.

## Patentansprüche

1. Phytotherapeutische Zusammensetzung,
**dadurch gekennzeichnet,**
**daß** sie als Wirkstoffe nur Extrakte von Morus nigra L. und Artemisia absinthium L. aufweist.

2. Phytotherapeutische Zusammensetzung nach Anspruch 1,
**dadurch gekennzeichnet,**
**daß** die Extrakte ausgehend von einem einzigen Gemisch erhalten werden können, das Morus nigra L. und Artemisia absinthium L. aufweist.

3. Phytotherapeutische Zusammensetzung nach Anspruch 2,
**dadurch gekennzeichnet,**
**daß** das Gemisch Morus nigra L. mit einem Anteil von 52 bis 63 Gew.-% und Artemisia absinthium L. mit einem Anteil von 37 bis 48 Gew.-% aufweist.

4. Zusammensetzung nach Anspruch 3,
**dadurch gekennzeichnet,**
**daß** das Gemisch Morus nigra L. mit einem Anteil von 57 Gew.-% und Artemisia absinthium L. mit einem Anteil von 43 Gew.-% aufweist.

5. Phytotherapeutische Zusammensetzung nach einem
oder mehreren der vorstehenden Ansprüche,
**dadurch gekennzeichnet,**
**daß** die Extrakte in einer hydroalkoholischen Lösung gelöst sind.

6. Phytotherapeutische Zusammensetzung nach Anspruch 5,
**dadurch gekennzeichnet,**
**daß** die hydroalkoholische Zusammensetzung eine Konzentration von 60 % hat.

7. Verfahren zum Herstellen einer phytotherapeutischen Zusammensetzung nach einem oder mehreren der vorstehenden Ansprüche, das die folgenden Schritte aufweist:
- Herstellen eines Gemischs, das Morus nigra L. und Artemisia absinthium L. aufweist;
- Aufschließen des Gemischs in einer hydroalkoholischen Lösung;
- Filtrieren des Gemischs.

8. Verfahren nach Anspruch 7,
**dadurch gekennzeichnet,**
**daß** beim Schritt zum Aufschließen das Gewichtsverhältnis zwischen dem Gemisch und der hydroalkoholischen Lösung 1:10 beträgt.

9. Verfahren nach Anspruch 7 oder 8,
**dadurch gekennzeichnet,**
**daß** der Schritt zum Aufschließen bei Raumtemperatur erfolgt.

10. Verfahren nach Anspruch 9,
**dadurch gekennzeichnet,**
**daß** die Dauer des Schritts zum Aufschließen mindestens 2 Wochen beträgt.

## Revendications

1. Composition phytothérapeutique **caractérisée en ce qu'**elle comprend uniquement en tant que ingrédient actif des extraits de Morus Nigra L et d'Arthémisia Absentium L.

2. Composition phytothérapeutique selon la revendication 1, **caractérisé en ce que** lesdits extraits peuvent être obtenus en partant d'un simple mélange comprenant Morus Nigra L et Arthémisia Absentium L.

3. Composition phytothérapeutique selon la revendication 2, **caractérisé en ce que** ledit mélange comprend entre 52% et 53% en poids de Morus Nigra L et entre 37% et 48% en poids de Arthémisia Absentium L.

4. Composition phytothérapeutique selon la revendication 3, **caractérisé en ce que** ledit mélange comprend 57% en poids de Morus Nigra L et 43% en poids d'Arthémisia Absentium L.

5. Composition phytothérapeutique selon une ou plusieurs des revendications précédentes, **caractérisé en ce que** lesdits extraits sont dissouts dans une solution hydroalcoolique.

6. Composition phytothérapeutique selon la revendication 5, **caractérisé en ce que** ladite composition hydroalcoolique présente une concentration de 60%.

7. Procédé pour la production d'une composition phythothérapeutique selon l'une ou plusieurs des revendications précédentes, comprenant les étapes de :
- préparer un mélange comprenant Morus Nigra L et Arthémisia Absentium L ;
- faire macérer ledit mélange dans une solution hydroalcoolique ;
- filtrer le mélange.

8. Procédé selon la revendication 7, **caractérisé en ce que** dans ladite étape de macération, le rapport de, poids entre ledit mélange et ladite solution hydroalcoolique est de 1:10.

9. Procédé selon la revendication 7 ou 8, **caractérisé en ce que** ladite étape de macération est effectuée à température ambiante.

10. Procédé selon la revendication 9, **caractérisé en ce que** la durée de l'étape de macération est d'au moins deux semaines.
